# EUROPÄISCHE PATENTANMELDUNG

(11) **EP 1 743 753 A1**
(43) Veröffentlichungstag der Anmeldung: **17.01.2007**
(21) Anmeldenummer: 06012146.4
(22) Anmeldetag: 13.06.2006
(51) Int. Cl.: B28B 7/38, A61K 6/00

(54) **Formgebungspaste, ihre Verwendung sowie Verfahren zur Herstellung eines keramischen Körpers**

(30) Priorität: 20.06.2005 DE 10528721
(71) Anmelder: Heraeus Kulzer GmbH, 63450 Hanau (DE)
(72) Erfinder: Hühn, Robin, 63517 Rodenbach (DE); Koops, Ulrich, Dr., 64380 Rossdorf (DE); Weiss, Özlem, Dr., 65929 Frankfurt (DE)
(74) Vertreter: Kühn, Hans-Christian

(57) **Zusammenfassung**

Eine Formgebungspaste zur Anwendung in der keramischen Formgebung enthaltend mindestens ein Trennmittel, mindestens ein Ionen abgebendes Mittel und/oder oder eine amphotere Substanz hat insbesondere eine elektrostatisch destabilisierende Wirkung auf Keramikschlicker.

## Beschreibung

Die Erfindung betrifft eine Zusammensetzung als Formgebungspaste zur Anwendung in der keramischen Formgebung sowie ihre Verwendung in keramischen Formgebungsverfahren ausgehend von keramischen Suspensionen.

Zur Herstellung keramischer Bauteile können nach dem Stand der Technik sehr unterschiedliche Verfahren angewendet werden. Diese können unterteilt werden in Pressverfahren, plastische Formgebung und Gießtechniken mit keramischen Suspensionen. Grundlage der Gießtechniken ist das Einbringen einer stabilen keramischen Suspension (ein sogenannter Schlicker) in eine poröse Form, meist aus Gips oder auch aus mikroporösem Kunststoff, die der Suspension durch Kapillarkräfte die Flüssigkeit entzieht, so dass sich an der Formenwand eine kompakte Teilchenschicht (Scherben- bzw. Formkörperbildung) bildet. Der erhaltene sogenannte Grünkörper muss anschließend zur endgültigen Verdichtung gesintert werden und schwindet dabei um bis zu 30 %, welches bei der Auslegung der Gipsform berücksichtigt werden muss. Ein großer Vorteil dieses Verfahrens ist die Möglichkeit auch komplizierte Teile hinsichtlich Dünnwandigkeit und unsymmetrischer Form herstellen zu können. Dabei kann das Gießverfahren soweit automatisiert werden, dass auch Kleinserien wirtschaftlich herstellbar sind. Nachteilig und begrenzend sind die langen Zeiten der Scherbenbildung, der kritische Vorgang der Entnahme des Formkörpers und die geringe Haltbarkeit der Formen, die nach jedem Formgebungsvorgang lange getrocknet werden müssen und somit stark beansprucht werden.

Eine technische Lösung der Größenanpassung von der Form auf den Schwindungsgrad des Grünkörpers und das kritische Ablösen von der Form wurde in der Dentaltechnik in der EP 0 241 384 durch ein Verfahren gelöst, in dem auf einem Duplikat des Zahnstumpfmodells aus einem Spezialgips der Schlicker mit einem Pinsel schichtweise aufgetragen wird oder durch mehrmaliges Eintauchen des Stumpfs in den Schlicker an dem Stumpf sich eine Kappe bildet, die bereits ihre endgültige Dimension aufweist. Dieses Verfahren dient vor allem zur Herstellung von Kronengerüsten. Anschließend folgt ein erster Brand bei niedrigen Temperaturen während sich der Grünkörper noch auf dem Stumpf befindet. Dabei löst sich das Gipsmodell durch den geringen Schrumpf von bis zu 2 % langsam vom Grünkörper ab. Aus dem Grünköper wird im anschließenden ersten Sinterbrand ein poröser Weißling gebildet, der im letzten Schritt durch eine Glasinfiltration seine endgültige Festigkeit erhält. Nachteile dieses Prozesses sind die langen Verarbeitungszeiten durch Duplikation des Meistermodells, die nicht kontrollierbare Schichtstärke der Kappe und die Fehleranfälligkeit durch die rein manuelle Verarbeitung.

Eine Weiterentwicklung der Formgebung aus keramischen Schlickern ist die elektrophoretische Abscheidung an einer leitfähigen Form. Dabei wird eine leitfähige Schicht auf die Form aufgebracht und durch Anlegen einer Gleichspannung wandern die in der Umgebung befindlichen geladenen Schlickespartikel an die Form und scheiden sich dort ab (DE 103 32 802 A1, DE 103 46 775 A1, DE 103 39 603 A1). Eine weitere Maßnahme, die dem Erzeugen einer gleichmäßigen Schichtdicke durch Entwässern dienen soll, ist laut DE 101 27 144 B4 das Auftragen einer hygroskopischen Schicht, etwa eines Gels. Optional kann gemäß Anspruch 4 eine Spannung angelegt werden, um den Niederschlag von Teilchen zu beschleunigen, womit die Formgebung ebenfalls auf dem Prinzip der Elektrophorese beruht. In der Praxis hat sich gezeigt, dass dieses Verfahren ohne Anlegen einer Gleichspannung das mehrmalige Tauchen der Form in den Schlickers erfordert, wobei die entstehende Schichtstärke nicht kontrolliert werden kann. Dies wird dadurch gelöst, dass die entstandene Form anschließend auf die gewünschte Stärke zurückgefräst wird. Eine gleichmäßige Schichtstärke entsteht nur durch das Prinzip der Elektrophorese. Die Nachteile der Elektrophorese sind die zwingend notwendige Leitfähigkeit der Form, an der abgeschieden wird, d.h die Gipsform muss mit einer leitfähigen Schicht versehen werden, oder es muss mit einem leitfähigen Formmaterial gearbeitet werden. In beiden Fällen sind nach der Herstellung der Gipsform weitere manuelle Prozessschritte notwendig, die einerseits den Gesamtprozess verlängern und andererseits Fehlerquellen darstellen.

Die Aufgabe war es, die keramische Formgebung zur Herstellung geometrisch anspruchsvoller Endkörper wie z.B. Kronenkappen dahingehend zu verbessern, dass der Prozess zeitlich erheblich beschleunigt und die Schichtstärke ohne das Prinzip der Elektrophorese kontrollierbar wird.

Es wurde gefunden, dass die Herstellung keramische Formkörper gezielt hinsichtlich der gewünschten Schichtstärke steuerbar ist und alternative Formmaterialien zu Gipsen oder porösen Kunststoffen wie z.B. Glas, verschiedene Kunststoffe oder Metalle verwendet werden können, wenn die Formgebung durch den Kontakt des Schlickers mit Formgebungspaste gemäß Anspruch 1 erfolgt.

Voraussetzung zur elektrostatischen Destabilisierung zur Induktion der kontrollierten Koagulation ist ein elektrostatisch stabilisierter Schlicker. Die Stabilisierung kann des Schlickers unter Einbeziehung des Zeta-Potentials und des isoelektrischen Punkts (IEP) gezielt eingestellt werden. Zum Beispiel haben Aluminiumoxide aus dem Bayer-Prozess einen sehr breiten IEP, so dass ein rein wässriger Schlicker bereits genügend stabilisiert sein kann. Die elektrostatische Stabilisierung führt im Schlicker zu einer losen Anordnung im flüssigen Milieu. Durch Veränderung der Abstoßungskräfte (Veränderung der Ladungsausbreitung der lonenhülle eines keramischen Teilchens, elektrostatische Destabilisierung) kann die lose Anordnung im flüssigen Milieu aufgehoben werden und eine gezielte Kompaktierung des im Schlicker enthaltenen Keramikpulvers erreicht werden.

Die Erfindung betrifft daher in einer Ausführungsform Formgebungspaste zur Anwendung in der keramischen Formgebung enthaltend
- mindestens ein Trennmittel und
- mindestens ein lonen abgebendes Mittel und/oder eine amphotere Substanz.

Bevorzugt stellt die Paste eine elektrostatisch destabilisierende Zusammensetzung dar.

Das Trennmittel gehört vorzugsweise der Gruppe gebildet aus Cremes, Gels, Salben oder Reinigungsmitteln an. Das lonen abgebende Mittel ist bevorzugt ein wasserlösliches Salz, besonders bevorzugt aus der Gruppe gebildet aus Alkali-, Ammonium- oder Erdalkalihalogeniden. Die amphotere Substanz ist bevorzugt ein Detergens wie z.B. SDS oder Tween. Zusätzlich kann ein wässriges Lösungsmittel und ein Emulgator vorhanden sein. Das Salz liegt zu etwa 3-80 Gew.% in der Zusammensetzung vor, vorzugsweise zu 5-70 Gew.%, insbesondere zu 7-60 Gew.%.

Die Erfindung betrifft auch die Verwendung einer oben beschriebenen Formgebungspaste zur Herstellung keramischer Körper aus einem Keramikschlicker, besonders zur Herstellung von Zahnersatzteilen.

Die Erfindung betrifft auch ein Verfahren zur Herstellung keramischer Körper aus einem Keramikschlicker, wobei
**A** auf eine Oberfläche eines Modells eine Formgebungspaste enthaltend
   • mindestens ein Trennmittel und
   • mindestens ein lonen abgebendes Mittel und/oder eine amphotere Substanz, aufgebracht wird,
**B** danach die Formgebungspaste mit dem Keramikschlicker in Kontakt gebracht,
**C** der Körper durch Kompaktierung mindestens eines Teils des Keramikschlickers an der Formgebungspaste gebildet und
**D** danach von dem Modell entfernt wird.

Überraschenderweise hat sich gezeigt, dass ein derartiges Verfahren neben der Dentaltechnik auf weitere Anwendungsbereiche von Keramiken ausgeweitet werden kann. Mit diesem Verfahren ist es möglich, unabhängig von der Natur der Form durch eine Formgebungspaste keramische Formkörper herzustellen, die sich zudem leicht aus der Form entfernen lassen.

Aus Schlickern lassen sich somit unter gegebenen Voraussetzungen Bauteile durch gezielte Formgebung durch das Prinzip der elektrostatischen Destabilisierung herstellen.

Die Erfindung betrifft somit auch die Verwendung handelsüblicher Formulierungen wie Cremes, Gels, Salben oder Reinigungsmitteln als Formgebungspasten, die entweder bereits ausreichend hohe Salzgehalte oder Elektrolytgehalte haben oder die nachträglich mit einem Salz versehen werden.

Selbstverständlich können entsprechende Pasten auch durch Mischen der Komponenten hergestellt werden.

Die Erfindung betrifft auch ein Verfahren zur Destabilisierung elektrostatisch stabilisierter Schlicker durch Imprägnierung der Oberfläche des Modells, das beschichtet werden soll, mit einer Formgebungspaste enthaltend
eine Pastengrundlage
lösliche anorganische oder organische Salze, bevorzugt Alkali- und Erdalkalihalogenide, anorganische oder organische Ammoniumsalze.

Auch alle anderen löslichen Verbindungen, die die lonenhülle der Partikel verändern, führen zur Ausbildung einer geeigneten Schicht. Es kann z.B. auch ein Metallsalz wie CoSO₄ verwendet werden oder ionische oberflächenaktive Substanzen.

Ohne wiederum die Erfindung zu beschränken, erklärt man sich den Effekt folgendermaßen: Die enthaltenen lonen gehen bei Kontakt mit dem Schlicker in Lösung und verkleinern die Ladungsausbreitung der lonenhülle der keramischen Teilchen. Dadurch rücken diese Teilchen näher zusammen und verhaken sich zu einer kompakteren Schicht, die mechanisch stabil ist. Durch den Salzgehalt der Paste kann die Zeit zur Ausbildung der Schicht gesteuert werden. Ist die Paste dann außerdem auf Fettbasis formuliert, wird zusätzlich das Herauslösen oder Ablösen der gebildeten Form vom Modell erleichtert. Dadurch können gezielt Schichten auf Freiformen erzeugt werden. Anschließende Trocknung erzeugt poröse Schichten, die gesintert werden können.

In einer umfangreichen Studie konnte nachgewiesen werden, dass die Löslichkeit des Salzes eine entscheidende Rolle spielt. Für die Kompaktierung zur Formgebung müssen die Ionen im Schlicker verfügbar sein oder zumindest an der Grenzfläche der Formgebungspaste zum Schlicker in Kontakt mit den Partikel treten, um die diffuse elektrische Doppelschicht verändern zu können. Dies bestätigen Untersuchungen mit Salzen unterschiedlicher Löslichkeit.

### Beispiel 1: Formgebungspaste auf Basis handelsüblicher kosmetischer oder medizinischer Emulsionen, Gele und Cremes

Eine handelsübliche Hautschutzsalbe z.B. Tactosan (W/O-Emulsion, Stockhausen Hautschutz) wird bis zur Schmelze erhitzt, anschließend wird eine wässrige 60%ige Magnesiumchlorid-Lösung eingerührt und die ganze Mischung unter Rühren homogenisiert. Nach Abkühlen hat die Mischung eine pastenartige Konsistenz und ist bereit für die Formgebung. Die Konzentration der Magnesiumchlorid-Lösung lässt sich gegebenenfalls zwischen 3 und 80 Gew. % variieren. Alternativ zu einer W/O-Emulsion lässt sich auch eine medizinische Elektrodencreme verwenden, die ihrer Anwendung entsprechend bereits einen Salzgehalt enthält wie z.B. electroden creme (Fa. Marquette HELLIGE) mit 5 Gew% NaCl.

### Beispiel 2: Formgebungspasten mit den Einzelkomponenten

### Vaselinebasis:

5g Vaseline
1g Cetylpalmitat
2g Ammoniumacetat
1g Tween 20
1 g Wasser
sowie

### Paraffinbasis

7g Paraffin fl.
1,5g Wasser
3,2g Tween 20
1g CaCl₂
werden durch inniges Vermischen der Komponenten hergestellt.

### Beispielhafte Ausführung des Verfahrens:

Die zu beschichtende Form wird dünn mit einer Paste nach Beispiel 1 oder 2 bestrichen und je nach gewünschter Schichtdicke und Salzgehalt der Paste 1 bis 10 Minuten in einen Schlicker getaucht. Dabei ist die Abscheidung desto schneller, je höher der Salzgehalt ist. Bei einer Salzlösung von 60% MgCl₂ gemäß Beispiel 1 erhält man nach 2 Minuten eine Schichtstärke von ca. 670 µm. Nach Ende der Tauchzeit wird die Form aus dem Schlicker gezogen, kurz in einem Luftstrom bei 25-60°C getrocknet, und kann dann von der Form abgehoben und anschließend bei 1120°C gesintert werden.

## Patentansprüche

1. Formgebungspaste zur Anwendung in der keramischen Formgebung enthaltend
• mindestens ein Trennmittel,
• mindestens ein lonen abgebendes Mittel und/oder eine amphotere Substanz.

2. Elektrostatisch destabilisierende Zusammensetzung gemäß Anspruch 1.

3. Zusammensetzung nach Anspruch 1 oder 2, wobei das Trennmittel der Gruppe gebildet aus Cremes, Gels, Salben oder Reinigungsmitteln angehört.

4. Zusammensetzung nach einem der vorstehenden Ansprüche, wobei das lonen abgebende Mittel ein wasserlösliches Salz ist.

5. Zusammensetzung nach einem der vorstehenden Ansprüche, wobei die amphotere Substanz ein Detergens ist.

6. Zusammensetzung nach einem der vorstehenden Ansprüche, **dadurch gekennzeichnet, dass** sie zusätzlich ein wässriges Lösungsmittel und einen Emulgator enthält.

7. Zusammensetzung nach Anspruch 3, **dadurch gekennzeichnet, dass** das Salz der Gruppe gebildet aus Alkali-, Ammonium- oder Erdalkalihalogeniden angehört.

8. Zusammensetzung nach Anspruch 3 oder 6, **dadurch gekennzeichnet, dass** das Salz zu 3-80 Gew.% darin vorliegt.

9. Zusammensetzung nach Anspruch 7, **dadurch gekennzeichnet, dass** das Salz zu 5-70 Gew.% darin vorliegt.

10. Zusammensetzung nach Anspruch 8, **dadurch gekennzeichnet, dass** das Salz zu 7-60 Gew.% darin vorliegt.

11. Verwendung einer Formgebungspaste nach einem der vorstehenden Ansprüche zur Herstellung keramischer Körper aus einem Keramikschlicker.

12. Verwendung nach Anspruch 10 zur Herstellung eines Zahnersatzteils.

13. Verfahren zur Herstellung keramischer Körper aus einem Keramikschlicker, wobei
**A** auf eine Oberfläche eines Modells eine Formgebungspaste gemäß Anspruch 1 aufgebracht wird,
**B** danach die Formgebungspaste mit dem Keramikschlicker in Kontakt gebracht,
**C** der Körper durch Kompaktierung mindestens eines Teils des Keramikschlickers an der Formgebungspaste gebildet und
**D** danach von dem Modell entfernt wird.
